# EUROPÄISCHE PATENTSCHRIFT

(11) **EP 1 083 985 B1**
(45) Veröffentlichungstag und Bekanntmachung des Hinweises auf die Patenterteilung: **17.09.2003**
(21) Anmeldenummer: 00931015.2
(22) Anmeldetag: 07.04.2000
(51) Int. Cl.: B01F 9/00, A61F 2/06, C12M 3/04

(54) **ROLLHALTERUNG**
ROLLING MOUNT
SUPPORT ROTATIF

(30) Priorität: 07.04.1999 DE 19915611
(43) Veröffentlichungstag der Anmeldung: 21.03.2001
(73) Patentinhaber: Bader, Augustinus, 31275 Immensen (DE)
(72) Erfinder: Bader, Augustinus, 31275 Immensen (DE)
(74) Vertreter: Bösl, Raphael, Dr. rer. nat., Dipl.-Chem.
(86) Internationale Anmeldenummer: DE0001062
(87) Internationale Veröffentlichungsnummer: WO00061271

(56) Entgegenhaltungen:
- WO-A-92/22634
- DE-U- 9 112 738
- FR-A- 963 775
- GB-A- 2 002 814
- US-A- 5 380 662

## Beschreibung

Die Erfindung betrifft eine Halterung für wenigstens einen Versorgungsanschlüsse aufweisenden Reaktor zum Durchführen einer Behandlung unter Drehen oder Rollen des Reaktors. Als Reaktor kann ein beliebiges Gefäß, wie z. B. eine Flasche, eine Rollflasche, verschlossene Reagenzröhrchen oder dergleichen dienen.

Insbesondere ist die Halterung für die Behandlung von Rollflaschen, Zellkultur- oder Reaktionsgefäßen, aber auch von Bioreaktoren, in einem Rollschrank oder einem Raum mit Rollvorrichtungen geeignet.

Im Stand der Technik sind Rollflaschen bekannt, die speziell für das Einlegen in einen sogenannten Rollschrank vorgesehen sind, in welchem die Rollflaschen unter Einhaltung bestimmter äußerer Bedingungen kontinuierlich durch Walzen bewegt, nämlich gerollt werden. Hierdurch wird der Inhalt der Rollflaschen in ständiger drehender Bewegung gehalten. Im allgemeinen kann der Rollschrank temperiert werden, so dass das zu behandelnde Behälterinnere bei einer konstanten, z. b. physiologischen Temperatur gehalten werden kann.

In einem Rollschrank erfolgt die Drehung der darin eingelegten Flasche stets um ihre Längsachse. Durch eine solche Rollbewegung ist es daher nicht möglich, den Flaschen- bzw. Behälterinhalt in der Flasche durch das Rollen wirklich gleichmäßig zu verteilen oder zu vermischen. Diese gilt auch besonders für Reaktoren wie Bioreaktoren, da dort das Vermischen durch Einbauten zusätzlich erschwert sein kann.

WO 92/22534 offenbart eine Rollflasche zum Herstellen von monoglonalen Antikörpern mit einem Behälter für ein Wuchsmedium, einem an und in dem Behälter befestigten abgedichteten Dialyserohr, so dass es in das Wuchsmedium eingetaucht sein kann, und mit Mitteln um dem Behälter eine solche Bewegung zu erteilen, dass bei einer zum Belassen einer Blase ausreichenden Füllung des Dialyserohres mit einer Kultur aus Hybridomazellen die Blase in eine entlang des Rohres hin- und hergehende Schwingung versetzt wird, um die Kultur aus Hybridomazellen in Suspension zu halten.

GB-A-2 002 814 offenbart eine Vorrichtung zur Züchtung von tierischen und humanen Gewebezellen in einzelliger Schicht und zur Vermehrung von Viren auf diesen Gewebekulturen mit einem mit Zu- und Abläufen für Substratflüssigkeit und sauerstoffhaltige Gase versehenen, insbesondere zylindrischen Behälter mit einem in diesem mit einer Welle gelagerten rotierbaren Innenteil mit einer Vielzahl von Rohren als Gewebezuchtflächen. Die Rohre sind beidseitig offen und etwa parallel zur Welle in auf dieser aufgebrachten gelochten Haltescheiben lösbar gehalten.

Der Erfindung liegt das Problem zugrunde, eine Vorrichtung bereitzustellen, mit Hilfe derer Behälter, Flaschen, Reaktoren und dergleichen, die in einem Rollschrank behandelt werden sollen, auf einfache Weise mit einem Fluid versorgt werden können. Während der Behandlung in dem Rollschrank soll dabei eine kontinuierliche Versorgung mit dem Fluid gewährleistet sein.

Diese Aufgabe wird durch eine Halterung gemäß Anspruch 1 gelöst. Die abhängigen Ansprüche betreffend vorteilhafte Ausgestaltungen der Erfindung.

Der Behälter oder Reaktor kann jeweils innerhalb der Aufnahme(n) mit geeigneten Befestigungsmitteln befestigt, oder auch nur eingeschoben oder eingesteckt sein, wobei die Reibungskraft für sicheren Halt sorgt.

Die rollfähige äußere Form kann auch bei der alternativen Ausgestaltung der Halterung vorzugsweise eine Zylinderform, eine Kugel- oder Ballform sein. Wenn die äußere Form der Halterung zylinderförmig ist, ist es insbesondere vorteilhaft, wenn die Hauptlängsachse der Aufnahme in einem Winkel zu einer Drehachse der rollfähigen Halterung steht. Die Aufnahme ist dann vorzugsweise so angeordnet, dass der eingesetzte Behälter schräg zur Längsachse des Zylinders steht, um die die Drehung im Drehschrank erfolgt. Hierdurch wird die auch auf den Behälter ausgeübte Drehbewegung mit einer Schwenkbewegung vom einen zum anderen Ende des eingesetzten Behälters überlagert, sobald der Behälter in der Halterung in einem Rollschrank gerollt wird.

Es können verschiedene Winkel zwischen 0 und 90° zwischen der Achse der Aufnahme und damit des eingesetzten Behälters und der (Haupt-)Rollachse der rollfähigen Halterung verwirklicht werden.

Die Halterung kann auch ungleichmäßig geformt sein, sofern sie rollfähig ist. Wenn die Halterung kugel- oder ballförmig ist, wird sich in dem Rollschrank eine zufallsgesteuerte Bewegung um verschiedene Raumachsen einstellen. Die zufallsgesteuerte Bewegung kann durch außen an der ballförmigen Halterung angeformte Auslenknoppen weiter unterstützt werden. Anstelle der Auslenknoppen können - gleich an welcher Halterungsform - allgemein andere Profiliermengen vorgesehen sein. Im Falle einer zylinderförmigen Halterung sind beispielsweise exzentrische Ringe um den Zylindermantel denkbar.

Für die Aufnahme einer Flasche, z.B. einer konventionellen Rollflasche, wird die Aufnahme der Halterung vorzugsweise röhrenförmig sein. Die Aufnahme kann auch jede andere Form haben, die dann vorzugsweise an die äußere Form der aufzunehmenden Behälter oder Reaktoren angepaßt ist.

In ein und derselben Halterung können auch mehrere Aufnahmen für das Einsetzten mehrerer Behälter oder Reaktoren vorgesehen coin.

Die Halterung wird verwendet, um einen rollbaren Behälter, wie z.B. eine Rollflasche mit mehr Freiheitsgraden als nur um seine Längsachse zu drehen, oder um einen als solchen nicht rollbaren Behälter, wie z.B. einen kastenförmigen Behälter in einem Rollschrank überhaupt behandelt zu können. Sie wird auch verwendet um einen mit Versorgungsanschlüssen versehenen Reaktor innerhalb eines Rollschrankes oder einer Drehapparatur problemlos weiter versorgen zu können.

In Weiterbildung der Erfindung ist insbesondere vorgesehen, dass die Halterung eine Vorrichtung zum Heizen oder Kühlen oder Thermostatisieren des oder der Behälter oder Reaktoren umfasst. Diese Heizvorrichtungen können vom Fachmann in an sich bekannter Weise ausgebildet werden. Beispielsweise können innerhalb der Halterung Heizdrähte und oder Kühlschlangen angeordnet sein, insbesondere um die Aufnahmen für die Behälter oder Reaktoren herum, um diese zu heizen zu kühlen oder zu thermostatisieren. Für eine Thermostatisierung ist zusätzlich wenigstens ein Temperaturfühler und wenigstens eine Regeleinheit erforderlich, die mit der zugehörigen Energieversorgung ebenfalls innerhalb der Halterung untergebracht werden können.

Die Halterung ist mit einem Reservoir und einem Leitungssystem zur Versorgung eines Reaktors über Kupplungsanschlüsse ausgestattet. In bevorzugter Ausführungsform ist eine Pumpe für das Umpumpen des fluiden Mediums innerhalb des Leitungssystems zwischengeschaltet. Sind mehrere Reaktoren in einer Halterung untergebracht, können auch mehrere Reservoirs und Leitungssysteme, einschließlich Pumpen, vorgesehen sein.

Ferner kann in Weiterbildung der Erfindung je Halterung wenigstens eine Energieversorgung - z.B. in Form einer Batterie - für gegebenenfalls innerhalb der Halterung durchzuführende Heiz-, Pump- oder Drehoperationen mitenthalten sein.

Die Halterung kann aus jedem geeigneten Material, z.B. Glas, Kunststoff, Metall oder Kombinationen hieraus bestehen.

Im folgenden wird die Erfindung anhand von Ausführungsbeispielen näher erläutert:
- Fig. 1: zeigt eine Halterung für einen zylindrischen Behälter, z.B. eine Kulturflasche, ein Reagenzröhrchen u.ä.;
- Fig. 2: zeigt eine kugelförmige Halterung mit Auslenknoppen sowie einer röhrenförmigen Ausnehmung;
- Fig. 3: zeigt eine walzenförmige Halterung für mehrere zylindrische Behälter/Flaschen;
- Fig. 4: zeigt eine schematische Ansicht einer halterung mit einer Aufnahme für einen Reaktor im Längsschnitt;
- Fig. 5: zeigt eine Anordnung zum Rollen einer Halterung (Fig. 5a) und eine Anordnung zum Drehen einer Halterung in einem Gestell (Fig. 5b).

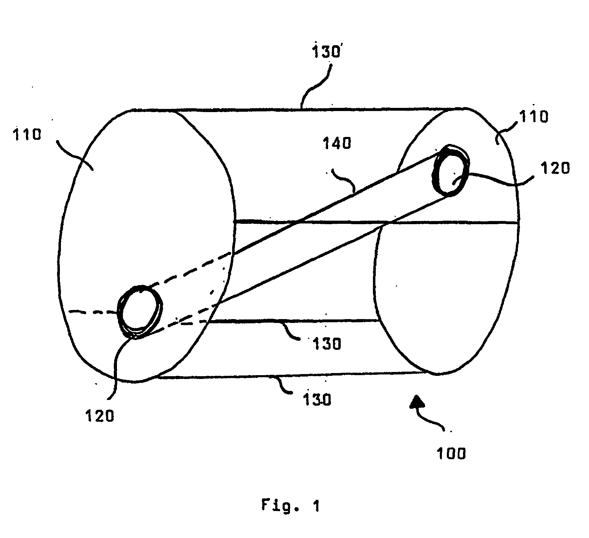

Figur 1 zeigt eine zylindrische rollbare Halterung 100, in die eine Kulturflasche oder ähnliches während einer Behandlung in einem Rollschrank eingesetzt werden kann. Die Halterung bewirkt, dass der eingesetzte Behälter zusätzlich zu der Rollbewegung einer Schwenkbewegung quer zu seiner Längsachse unterworfen wird. Die Halterung 100 besteht in diesem Ausführungsbeispiel aus zwei Scheiben 110 aus Glas oder Plexiglas, die azentrisch mit zwei ovalen (zylinderschnittförmigen) Öffnungen 120 versehen sind. Für den Einsatz eines zylindrischen Behälters sind die öffnungen 120 vorzugsweise oval, die Scheiben 110 sind in diesem Beispiel kreisförmig, könnten jedoch auch anders, z.B. eliptisch geformt sein. Die Scheiben 110 sind im vorliegenden Beispiel durch mehrere Stangen 130 verbunden, sie könnten jedoch auch durch einen geschlossenen Zylindermantel verbunden sein. Die Verbindung der Scheiben 110 ist so gewählt, dass sich die Öffnungen 120 in Projektion versetzt gegenüberliegen. Zwischen den Öffnungen 120 ist eine Röhre 140 eingesetzt, in die der jeweilige Behälter eingeschoben werden kann. Die Öffnungen 120 und die Röhre 140 bilden zusammen die Ausnehmung für den einzusetzenden Behälter. Die Längsachse des in der Zeichnung nicht dargestellten Behälters verläuft dann schräg zur Längsachse der insgesamt zylindrischen Halterung 100. Die Halterung 100 bildet mit dem Behälter zusammen eine Anordnung, die in einen Rollschrank eingelegt werden kann. Bei Behandlung im Rollschrank wird der Behälter einer Rollbewegung um seine Längsachse und gleichzeitig einer Schwenkbewegung quer zu seiner Längsachse ausgesetzt. Hierdurch kann der Behälterinhalt, z.B. ein Kulturmedium mit darin befindlichen Zellen, besonders intensiv und gleichmäßig verteilt, und beispielsweise mit einem ebenfalls in dem Behälter befindlichen Substrat in Kontakt gebracht werden.

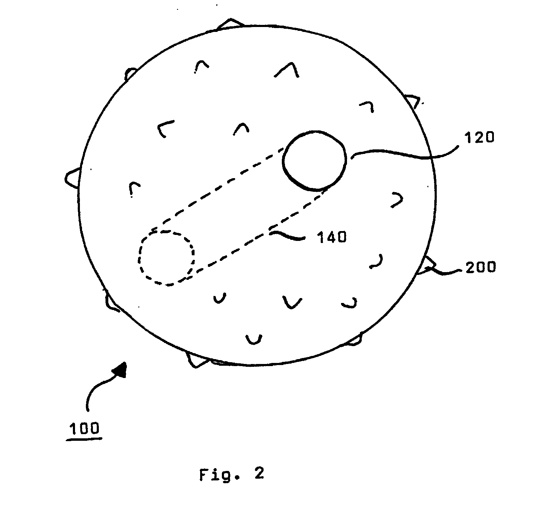

Figur 2 zeigt eine kugelförmige Halterung 100 mit zwei kreisförmigen Öffnungen 120 und einer darin eingesetzten durch die Kugelmitte verlaufenden Röhre 140. Die Röhre 140 bildet gemeinsam mit den öffnungen 120 eine Ausnehmung zum Einsetzen eines Behälters, der in einem Rollschrank in dieser Halterung 100 behandelt werden soll. Um die Bewegungsrichtung der Kugel im Rollschrank immer wieder nach dem Zufallsprinzip zu ändern, sind an der Außenwand der kugelförmigen Halterung 100 Auslenknoppen 200 angebracht. Hierdurch wird verhindert, dass sich beim Rollen eine Vorzugsrichtung einstellen kann. Die Kugel könnte beispielsweise auch aus einem Drahtgitternetz bestehen.

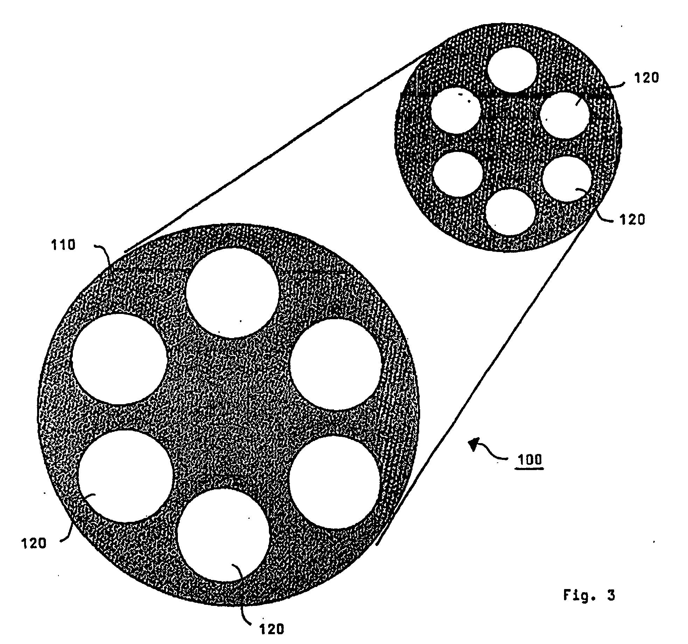

Figur 3 zeigt eine walzenförmige Halterung 100 für mehrere zylindrische Behälter, wie z.B. Rollflaschen, Reaktionsgefäße oder dergleichen. Diese Halterung 100 entspricht im Prinzip der zylindrischen Halterung aus Figur 1, mit dem Unterschied, dass in den beiden seitenbegrenzenden Scheiben 110 jeweils mehrere Öffnungen 120 vorgesehen sind, so dass mehrere längliche zylindrische Behälter - und zwar in diesem Ausführungsbeispiel maximal 6 Stück - in die Walze eingesteckt werden können. Die Behälter sollen dabei so in die Walze eingesteckt werden, dass ihre Achsen schräg zur Rollachse verlaufen, wenn die zusätzliche Schwenkbewegung quer zur Längsachse gewünscht ist, d.h. die beiden Enden eines zylindrischen Behälters werden in sich versetzt gegenüberliegende Öffnungen 120 eingeführt, ist dies nicht der Fall, können die Behälter auch parallel zur Rollachse eingesetzt werden.

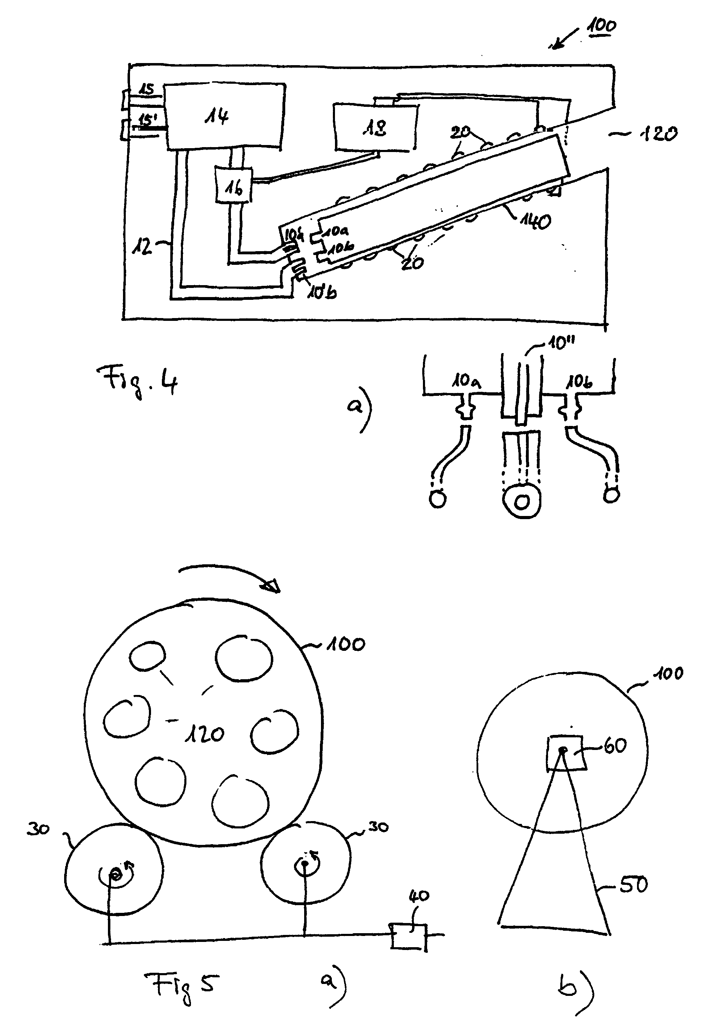

Figur 4 zeigt eine schematische Ansicht einer im Ganzen mit 100 bezeichneten Halterung im Längsschnitt. In dieser Zeichnung ist nur eine Aufnahme für einen Behälter oder Reaktor gezeigt, nämlich eine Röhre 140, die von einer Öffnung 120 her beschickt werden kann. Die Halterung 100 besteht aus einem äußerlich geschlossenen Zylinder, um die im folgenden noch näher beschriebenen enthaltenen Bauteile zu schützen. Innerhalb der röhrenförmigen Aufnahme 140 ist ein darin eingesetzter Reaktor A dargestellt, der Versorgungsanschlüsse 10a, 10b besitzt, beispielsweise Sterilkupplungen für Kulturmedium (Kulturmediumport). Den Versorgungsanschlüssen 10a, 10b liegen passende Kupplungsverbindungen 10'a, 10'b gegenüber, die mit einem Leitungssystem 12 verbunden sind, über das der Reaktor A versorgt wird, wenn die Versorgungsanschlüsse 10a, 10b und die zugehörigen Kupplungsanschlüsse 10'a, 10'b beispielsweise mittels einer Steckverbindung verbunden worden sind. Das Leitungssystem 12 steht mit einem Reservoir 14 zur Bevorratung des flüssigen oder gasförmigen Mediums in Verbindung, mit dem der Reaktor A versorgt werden soll. Das Reservoir 14 kann über die Anschlüsse 15, 15' gefüllt oder entleert werden. Es kann sich aus zwei getrennten Behältern für Frischmedium und Abfall zusammensetzen. In diesem Ausführungsbeispiel ist in dem Leitungssystem 12 eine Pumpe 16 zwischengeschaltet - beispielsweise eine Schlauchpumpe -, die zur Energieversorgung mit einer Batterie oder ein Akku 18 verbunden ist. Die Batterie 18 liefert hier gleichzeitig Strom für die Heizdrähte 20, die die Röhre 140 spiralförmig umgeben. Hierdurch kann die Aufnehmung, die den Reaktor A beherbergt, geheizt werden.

In dem in Figur 4 gezeigten Beispiel besitzt der Reaktor A zwei Anschlüsse, 10a und 10b, d.h. einen Zu- und einen Ablauf für flüssiges Versorgungsmedium. Es können jedoch mehr anschlüsse vorhanden sein, beispielsweise zusätzlich für N2/O2/CO2-Versorgungen. In die Halterung können noch andere Elemente als die in Figur 4 gezeigten integriert werden, beispielsweise Vorrichtungen für eine Kryokonservierung, ein Auftausystem oder ein oder mehrere Chips zum Überwachen und Steuern der Einzelfunktionen.

In der Detaillzeichnung nach Figur 4a ist schematisch der untere Abschnitt eines Reaktors mit drei Anschlüssen und prinzipieller Kupplungsmöglichkeit gezeigt, wobei die Anschlüsse 10a und 10b einfache Zu- und Abläufe sind, während Versorgungsanschluss 10'' ein Cooxialanschluss ist. Die Zuund Abströmverhältnisse sind unter den Anschlüssen dargestellt.

Figur 5 zeigt in Abbildung 5a eine Anordnung zum Rollen einer beliebigen Halterung und in Abbildung 5b eine Anordnung zum Drehen einer Halterung in einem Gestell.

Figur 5a zeigt eine beliebige zylinderförmige Halterung 100 mit hier sechs Ausnehmungs-Öffnungen 120 in Frontansicht. Die zylinderförmige Halterung 100 liegt auf Walzen 30 auf, die über einen Antrieb 40 mittels einer beliebigen Übertragung angetrieben und gedreht werden.

Figur 5b zeigt eine in diesem Beispiel ebenfalls zylinderförmige Halterung 100, deren Zylinderachse drehbar in ein Gestell 50 eingesetzt ist. Auf der Drehachse ist weiterhin ein schematisch dargestellter Drehantrieb 60 angeordnet, dessen weitere Anschlüsse nicht gezeigt sind. Die Halterung 100 gleicht ansonsten der in Figur 4 dargestellten.

## Patentansprüche

1. Halterung (100) für wenigstens einen Versorgungsanschlüsse aufweisenden Reaktor zum Durchführen einer Behandlung unter Drehen oder Rollen des Reaktors, wobei die Halterung entweder eine rollfähige äußere Form besitzt oder in einem Drehgestell drehbar aufgehängt und mit einem Drehantrieb versehen oder verbunden ist,
wobei die Halterung (100) umfasst:
- wenigstens eine Aufnahme (140) für den wenigstens einen Reaktor,
- ein Reservoir (14) zur Bevorratung eines flüssigen oder gasförmigen Mediums, und
- ein Leitungssystem (12) für die Versorgung des einen Reaktors oder der mehreren Reaktoren mit dem flüssigen oder gasförmigen Medium des Reservoirs (14), wobei das Leitungssystem (12) in Kupplungen (10'a, 10'b) mündet, die auf Versorgungsanschlüsse (10a, 10b) des Reaktors adaptiert und an der zugehörigen Aufnahme angeordnet sind.

2. Halterung nach Anspruch 1, **dadurch gekennzeichnet, dass** die rollfähige äußere Form, eine Kugel- oder Ballform oder eine Zylinderform ist.

3. Halterung nach Anspruch 2, **dadurch gekennzeichnet, dass** die Hauptlängsachse der Aufnahme in einem Winkel zu einer Drehachse der rollfähigen Halterung steht.

4. Halterung nach einem der vorhergehenden Ansprüche, **dadurch gekennzeichnet, dass** die Aufnahme oder die Aufnahmen röhrenförmig ist oder sind.

5. Halterung nach einem der vorhergehenden Ansprüche, **dadurch gekennzeichnet, dass** auf einer abrollenden Fläche oder Kante der Halterung Auslenknoppen angeordnet sind.

6. Halterung nach einem der vorhergehenden Ansprüche, **dadurch gekennzeichnet, dass** an einem abrollenden Außenmantel der Halterung exzentrische Ringe angeordnet sind.

7. Halterung nach einem der vorhergehenden Ansprüche, **dadurch gekennzeichnet, dass** sie eine Vorrichtung zum Heizen oder Kühlen oder Thermostatieren des oder der Behälter oder Reaktoren umfasst.

8. Halterung nach einem der vorhergehenden Ansprüche **dadurch gekennzeichnet, dass** sie eine Pumpe für das Umpumpen des flüssigen oder gasförmigen Mediums innerhalb der Reaktorversorgungsleitungen umfasst.

9. Halterung nach einem der vorhergehenden Ansprüche, **dadurch gekennzeichnet, dass** sie eine Energieversorgung für die gegebenenfalls durchzuführende Heiz-, Pump- oder Drehoperationen umfasst.

## Claims

1. Holder (100) for at least one reactor with supply connections for administering a treatment implying a turning or rolling movement of the reactor, said holder either having a rolling-type outer shape or being rotatably suspended in a turning frame and provided or connected with a turning drive,
said holder (100) comprising:
- at least one receiver (140) for said at least one reactor,
- a tank (14) for storing a liquid or gaseous fluid, and
- a supply system (12) for feeding the liquid or gaseous fluid from the tank (14) to said reactor or several reactors, said supply system (12) ending up in couplers (10'a, 10'b) adapted to the supply connections (10a, 10b) of the reactor and located at the appropriate receivers.

2. Holder as defined in claim 1, **characterized in that** the rolling-type outer shape is a globe or ball or cylinder shape.

3. Holder as defined in claim 2, **characterized in that** the major longitudinal axis of the receiver forms an angle with a turning axis of the rolling-type holder.

4. Holder as defined in any of the preceding claims, **characterized in that** the receiver(s) is or are of tubular shape.

5. Holder as defined in any of the preceding claims, **characterized in that** deflection knobs are arranged at a rolling surface or edge of the holder.

6. Holder as defined in any of the preceding claims, **characterized in that** eccentric rings are arranged at a rolling outer jacket of the holder.

7. Holder as defined in any of the preceding claims, **characterized in that** it comprises a device for heating or cooling or thermostating the tank(s) or reactor(s).

8. Holder as defined in any of the preceding claims, **characterized in that** it comprises a pump for transferring the liquid or gaseous fluid from and to within the supply lines of the reactor.

9. Holder as defined in any of the preceding claims, **characterized in that** it comprises an energy supply for the heating, pumping or turning operations where required.

## Revendications

1. Fixation (100) pour au moins un réacteur disposant de plusieurs raccordements d'alimentation et destiné à un traitement dispensé dans un mouvement tournant ou de roulement dudit réacteur, la fixation soit possédant une forme extérieure permettant le roulement, soit étant suspendue de manière rotative dans un bâti tournant et pourvue d'un actionneur rotatif ou reliée à un tel actionneur,
la fixation (100) comprenant:
- au moins un logement (140) pour ledit réacteur au nombre d'au moins un,
- un réservoir (14) pour le stockage d'un fluide liquide ou gazeux, et
- un système de conduites (12) pour l'alimentation d'un réacteur ou de plusieurs réacteurs en ledit fluide liquide ou gazeux stocké dans le réservoir (14), le système de conduites (12) aboutissant à des raccords (10'a, 10'b) adaptés aux raccordements d'alimentation (10a, 10b) du réacteur et disposés au logement correspondant.

2. Fixation selon la revendication 1, **caractérisée en ce que** la forme extérieure permettant le roulement est une forme sphérique ou cylindrique.

3. Fixation selon la revendication 2, **caractérisée en ce que** l'axe longitudinal principal du logement est disposé de manière à former un angle par rapport à un axe tournant de ladite fixation permettant le mouvement de roulement.

4. Fixation selon l'une des revendications 1 à 3, **caractérisée en ce que** le logement ou les logements est ou sont tubulaire(s).

5. Fixation selon l'une des revendications 1 à 4, **caractérisée en ce que** des boutons déflecteurs sont disposés sur une surface ou arête de roulement de la fixation.

6. Fixation selon l'une des revendications 1 à 5, **caractérisée en ce que** des bagues excentriques sont disposées sur une enveloppe extérieure de roulement de la fixation.

7. Fixation selon l'une des revendications 1 à 6, **caractérisée en ce qu'**elle comprend un dispositif pour le chauffage ou le refroidissement ou le réglage thermostaté du ou des réservoir(s) ou réacteur(s).

8. Fixation selon l'une des revendications 1 à 7, **caractérisée en ce qu'**elle comprend une pompe pour le transvasement du fluide liquide ou gazeux à l'intérieur du réseau d'alimentation du ou des réacteur(s).

9. Fixation selon l'une des revendications 1 à 8, **caractérisée en ce qu'**elle comprend une alimentation énergétique pour les opérations de chauffage, de pompage ou de rotation à réaliser selon le cas.
